# EUROPÄISCHE PATENTSCHRIFT

(11) **EP 2 240 543 B1**
(45) Veröffentlichungstag und Bekanntmachung des Hinweises auf die Patenterteilung: **29.08.2012**
(21) Anmeldenummer: 09708255.6
(22) Anmeldetag: 03.02.2009
(51) Int. Cl.: C08L 35/06, C08L 33/08, C08L 71/02, C08F 8/14, C08F 8/44, C09D 135/06, C09D 133/08, C09D 171/02, C09D 17/00, C09D 11/00, C09D 7/02, A61K 8/81, A61Q 1/00, A61Q 3/02, A61Q 5/06, A61Q 17/04, C08G 65/332, C09B 67/00

(54) **NETZ- UND DISPERGIERMITTEL**
WETTING AND DISPERSING AGENT
AGENT MOUILLANT ET DISPERSANT

(30) Priorität: 04.02.2008 DE 102008007713
(43) Veröffentlichungstag der Anmeldung: 20.10.2010
(73) Patentinhaber: BYK-Chemie GmbH, 46483 Wesel (DE)
(72) Erfinder: GÖBELT, Bernd, 46487 Wesel (DE); NAGEL, Carsten, 48249 Dülmen (DE); OMEIS, Jürgen, 46286 Dorsten-Lembeck (DE); MEICHSNER, Marcus, 47475 Kamp-Lintfort (DE); WALTER, Diana, 46342 Velen (DE)
(74) Vertreter: Kutzenberger, Helga
(86) Internationale Anmeldenummer: PCT/EP2009/000699
(87) Internationale Veröffentlichungsnummer: WO 2009/098025

(56) Entgegenhaltungen:
- EP-A- 0 270 126
- EP-A- 1 640 389

## Beschreibung

Die vorliegende Erfindung betrifft an flüchtigen organischen Verbindungen (VOC)-arme Mischungen aus zumindest teilweise versalzten Copolymeren aus wenigstens einem ethylenisch ungesättigten, phenylgruppenhaltigen Monomeren und wenigstens einer α,β-ungesättigten Monocarbonsäure und/oder wenigstens einer α,β-ungesättigten Dicarbonsäure, aus wenigstens einem wasserlöslichen Polyether, Veresterungsprodukte aus wenigstens einem wasserlöslichen Polyether und einer aliphatischen Dicarbonsäure und aus einem Stempolymeren erhältlich durch Veresterung einer wenigstens 3 Carboxylgruppen aufweisenden Carbonsäure mit wenigstens einem wasserlöslichen Polyether sowie die Verwendung einer erfindungsgemäßen Mischung als Netz- und Dispergiermittel, vorzugsweise zur Herstellung von VOC-armen Pigmentpasten oder VOC-armen Lacksystemen.

Nach entsprechend verschärften Umweltrichtlinien werden in zunehmendem Maße nun mehr VOC-arme Lacke entwickelt. Infolgedessen werden sowohl bei Maler- und Bautenlacken als auch bei Automobillacken zur Beschichtung bzw. Lackierung hauptsächlich nur VOC-arme Systeme eingesetzt, deren Anteil an leicht flüchtigen, organischen Lösungsmittel (VOC = volatile organic compounds) nicht die nach ISO 11890-2 bzw. DIN 55649 festgelegten Grenzwerte überschreiten darf. Diese Definition steht im Weiteren für den Begriff VOC-arm.

Nur wenn die Werte an leicht flüchtigen, organischen Lösungsmitteln unterhalb dieser in den genannten ISO- und DIN-Normen festgelegten Grenzwerten liegen, können entsprechende Systeme als VOC-arm eingestuft werden. Dies hat zur Folge, dass es weiterhin einen dringenden Bedarf für Netz- und Dispergiermittel gibt, die geeignet sind, Pigmente unterschiedlichster Art so zu dispergieren, dass daraus hergestellte, wässrige VOC-arme Pigmentkonzentrate die notwendige Lagerstabilität und die notwendigen rheologischen Eigenschaften sowohl in Pigmentpasten als auch in entsprechenden Lacksystemen gewährleisten. Dieser Bedarf besteht sowohl für den Einsatz von anorganischen als auch von organischen Pigmenten.

Viele bekannte Netz- und Dispergiermittel sind aber nicht so universell einsetzbar, da sie nur die Herstellung von wässrigen Dispersionen einer bestimmten Art von Pigmenten gewährleisten.

So wird in US-A-4243430 die Verwendung einer Kombination aus Carboxylgruppen aufweisenden Copolymeren, die aus ethylenisch ungesättigten Monomeren hergestellt worden sind und als Ammonium und Zinksalze vorliegen, und nicht ionischen oder anionischen, polyetherhaltigen Benetzungsmitteln wie z. B. ethoxylierten Fettsäuren als Netz- und Dispergiermittel beschrieben. Der Einsatz von Zinkionen bzw. Zinkkomplexen in Netz- und Dispergiermitteln ist aus Umweltgründen nicht akzeptabel.

In US-A-6242499 wird die Verwendung von Partialestern von polyfunktionellen Carbonsäuren mit Polyglycidolen als Netz- und Dispergiermittel für nanoskalige Feststoffe in kosmetischen Zubereitungen offenbart.

In EP-A-1640389 werden ggf. versalzte Carboxylgruppen aufweisende Copolymerisate aus ethylenisch ungesättigten Dicarbonsäuren, ungesättigten Monocarbonsäurederivaten und α, β ungesättigten Polyethern beschrieben, die sich als die polymere Grundlage für Netz- und Dispergiermittel eignen.

Als Netz- und Dispergiermittel für Tinten wird gemäß US-A-6683121 eine Kombination aus carboxylhaltigen Copolymeren, die aus ethylenisch ungesättigten Monomeren aufgebaut sind, und Blockcopolymeren, die aus Ethylenoxid und Propylenoxid aufgebaut sind und einen HLB-Wert zwischen 16 und 32 aufweisen, empfohlen.

Gegenüber diesem Stande der Technik bestand die Aufgabe, ein VOC-armes Netzund Dispergiermittel zur Verfügung zu stellen, mit dem wässrige Konzentrate sowohl von anorganischen als auch von organischen Pigmenten hergestellt werden können, die eine ausgezeichnete Lagerstabilität und sehr gute rheologische Eigenschaften aufweisen, so dass sie sich problemlos zur Weiterverarbeitung in wässrigen Überzugsmitteln oder Universal-Tönpasten-Systemen eignen.

Diese Aufgabe wird durch das zur Verfügung stellen der erfindungsgemäßen Netzund Dispergiermittel gelöst, die eine VOC-arme Mischung aus
I 10 bis 80 Gew% eines zumindestens zu 50 % versalzten Copolymeren aus polymerisierten Einheiten von wenigstens einem ethylenisch ungesättigten, phenylgruppenhaltigen Monomeren und wenigstens einer vorzugsweise aliphatischen, α,β-ungesättigten Monocarbonsäure und/oder wenigstens einer vorzugsweise aliphatischen, α,β-ungesättigten Dicarbonsäure und ggfs. deren Derivaten,
II 1 bis 30 Gew% wenigstens eines wasserlöslichen, eine endständige OH-Gruppe aufweisenden, linearen, vorzugsweise aliphatischen Polyethers, der zumindestens aus 25 mol-% aus Ethylenoxid-Einheiten aufgebaut ist,
III 5 bis 80 Gew% wenigstens eines Veresterungsprodukts aus einer aliphatischen Dicarbonsäure, die vorzugsweise ein zahlengemitteltes Molekulargewicht von 400 g/mol bis 1000 g/mol aufweist, und aus wenigstens einem wasserlöslichen Polyether der Komponente 11 mit vorzugsweise einem zahlengemittelten Molekulargewicht von ≤ 2000 g/mol oder einer Mischung von Polyethem, die mindestens einen wasserlöslichen Polyether der Komponente II mit vorzugsweise einem zahlengemittelten Molekulargewicht von ≤ 5 2000 g/mol aufweist,
   und
IV 3 bis 80 Gew-% mindestens eines Sternpolymeren erhältlich durch Veresterung von wenigstens einer drei bis fünf Carboxylgruppen aufweisenden, aliphatischen Carbonsäure, die vorzugsweise ein Molekulargewicht von 500 g/mol bis 1500 g/mol aufweist, mit wenigstens einem der wasserlöslichen Polyether der Komponente II mit vorzugsweise einem zahlengemittelten Molekulargewicht von ≤ 2000 g/mol oder einer Mischung von Polyethern, die mindestens einen wasserlöslichen Polyether der Komponente II mit vorzugsweise einem zahlengemittelten Molekulargewicht von ≤ 2000 g/mol aufweist,
   wobei die Gew% der Komponenten I-IV jeweils auf das Gesamtgewicht der Komponenten I-IV bezogen sind und
   wobei die Summe der Gew-% der Komponenten I bis IV immer 100 Gew-% ergeben muss,
aufweisen.

Vorzugsweise weist die erfindungsgemäße VOC-arme Mischung
30 bis 70 Gew-% der Komponente I,
2 bis 10 Gew-% der Komponente II,
10 bis 50 Gew-% der Komponente III
   und
5 bis 20 Gew-% der Komponente IV
auf,
wobei die Summe der Gew-% der Komponente I bis IV immer 100 Gew-% ergeben muss und die Gew% der Komponenten I-IV jeweils auf das Gesamtgewicht der Komponenten I-IV bezogen sind.

Die Komponente I der Mischung umfasst ein zumindest zu 50 %, vorzugsweise zumindest zu 75 % versalztes, lineares Copolymeres, dass durch Polymerisation von wenigstens einem ethylenisch ungesättigten Monomeren vorzugsweise mit 8-20 C-Atomen, das mit mindestens einer Phenylgruppe substituiert ist, und wenigstens einer α,β-ungesättigten, vorzugsweise aliphatischen Monocarbonsäure mit vorzugsweise 3-8 C-Atomen und/oder aus wenigstens einer α,β-ungesättigten, vorzugsweise aliphatischen Dicarbonsäure mit vorzugsweise 4-10 C-Atomen und/oder deren cyclischen Anhydriden und ggfs. deren jeweiligen Derivaten, wie Ester und Amide, hergestellt wurde. Das zahlengemittelte Molekulargewicht dieser Copolymere beträgt 1.000 g/mol bis 20.000 g/mol, vorzugsweise 1.500 g/mol bis 10.000 g/mol.

Als vorzugsweise aliphatische, α,β-ungesättigte Mono- oder Dicarbonsäure werden vorzugsweise Monocarbonsäuren mit 3 bis 8 Kohlenstoffatomen, besonders bevorzugt Acrylsäure und/oder Methacrylsäure, und/oder α,β-ungesättigte Dicarbonsäure mit 4 bis 10 Kohlenstoffatomen und/oder deren cyclische Anhydride, besonders bevorzugt Maleinsäure, Fumarsäure, Itakonsäure und/oder Maleinsäureanhydrid, und ggf. deren jeweilige Derivate, besonders bevorzugt deren Ester, Amide, ganz besonders bevorzugt C₁- bis C₆-Alkylacrylate und/oder C₁- bis C₆-Alkylmethacrylate, eingesetzt.

Als phenylgruppenhaltige, ethylenisch ungesättigte Monomere werden vorzugsweise einfach ethylenisch ungesättigte Monomere mit 8 bis 20 C-Atomen, besonders bevorzugt Styrol, Aryl(meth)acrylate wie Benzyl(meth)acrylat, Phenylacrylat, wobei die Phenylreste ggfs. 1- bis 4-fach substituiert sein können wie beispielsweise 4-Methylphenyl(meth)acrylat. Ganz besonders bevorzugt wird ggfs. substituiertes Styrol eingesetzt.

Die Copolymeren der Komponente I können vorzugsweise aus 10 bis 90 Gew-%, phenylgruppenhaltigen, ethylenisch ungesättigten Monomeren und 90 bis 10 Gew-% aus α,β-ungesättigten Mono- und/oder Dicarbonsäuren aufgebaut sein.

Die Copolymeren der Komponente I können einen, statistischen, alternierenden, gradientenartigen oder blockartigen Aufbau besitzen. Copolymere mit einem gradientenartigen Aufbau sind beispielsweise in EP-1416019 sowie in WO 01/44389 beschrieben. Einem blockartigen Aufbau wie beispielsweise einem AB-, ABC- oder ABA-Blockaufbau können die Copolymeren der Komponente I gemäß Offenbarung in WO 00/40630, WO 03/046020, US-5085698, US-5160372, US-5519085, US-6849679 oder US 2007/0185272 erhalten.

Ganz besonders bevorzugte Copolymere der Komponente I sind Styrol/Maleinsäureanhydrid-Copolymerisate (SMA-Harze) mit einem Styrol-Maleinsäureanhydridverhältnis von 1:1 bis 8:1. Besonders bevorzugt 1:1 bis 2:1. lhr zahlengemitteltes Molekulargewicht entspricht den vorstehenden Angaben.

Die Copolymeren der Komponente I können über radikalisch initiierte Polymerisationen wie zum Beispiel mit Azo- oder Peroxidinitiatoren hergestellt werden. Um das gewünschte Molekulargewicht einzustellen, können Kettenregler wie zum Beispiel Thiole, sekundäre Alkohole oder Alkylhalogenide wie Tetrachlorkohlenstoff während der Polymerisation zugegeben werden. Als weitere Herstellverfahren für die Copolymeren, vorzugsweise SMA-Harze, können kontrollierte, radikalische Polymerisationsverfahren eingesetzt werden, wie zum Beispiel:
- der "Reversible Addition Fragmentation Chain Transfer Process" (RAFT), der bei Verwendung bestimmter Polymerisationsregler auch MADIX und Addition Fragmentation Chain Transfer genannt wird und vorliegend als RAFT bezeichnet wird, wie es beispielsweise in Polym. Int. 2000, 49. 993. Aust. J. Chem. 2005, 58, 379, J. Polym. Sci. Part A: Polym. Chem. 2005, 43, 5347, US 6 291 620, WO 98/01478, WO 98/58974 und WO 99/31144 beschrieben ist,
- die kontrollierte Polymerisation mit Nitroxylverbindungen als Polymerisationsregler (NMP), wie beispielsweise in Chem. Rev. 2001, 101, 3661 offenbart wurde.

Diese Offenbarungen in den genannten Referenzen werden hiermit als Teil der Offenbarung der vorliegenden Anmeldung eingeführt.

Die Copolymeren der Komponente I sind vorzugsweise zumindest zu 50 %, besonders bevorzugt zumindest zu 75 % versalzt, damit die Wasserlöslichkeit des Copolymeren erzielt wird. Für eine VOC-arme Versalzung der Copolymeren werden vorzugsweise Alkalimetall- oder Erdalkalimetall- Verbindungen, vorzugsweise entsprechende Hydroxide, Hydrogencarbonate oder Carbonate eingesetzt. Ganz besonders bevorzugt ist die Verwendung einer Alkalimetallverbindung, wie ein entsprechende Hydroxid, Hydrogencarbonat oder Carbonat. Insbesondere ganz besonders bevorzugt werden zur Versalzung Natrium- oder Kaliumhydroxid, Natrium-oder Kaliumhydrogencarbonat oder Natrium- oder Kaliumcarbonat verwendet.

Weiterhin ist es auch möglich, eine Versalzung der Copolymeren, insbesondere solche Copolymere mit Maleinsäureanhydridgruppierungen, durch Amidierung der Maleinsäure mit VOC-armen N,N-disubstituierten Diaminen unter Bildung von Amiden der Maleinsäureeinheit und einer zwitterionischen Struktur durch interne Versalzung der bei der Amidierung entstehenden Carboxylgruppen zu erreichen.

Für diese Art der Versalzung eigenen sich insbesondere N, N-disubstituierte Diamine der allgemeinen Formel R₂N-R'-NH₂ worin R für einen aliphatischen, cycloaliphatischen, aromatischen oder aliphatisch aromatischen Kohlenwasserstoffrest, vorzugsweise einen Alkylrest mit C₁ bis C₁₀, einen Cycloalkylrest mit C₄ bis C₈, einen Arylalkylenrest mit C₇ bis C₁₀ oder einen Arylrest mit C₆ bis C₁₂ steht. Bevorzugt steht der Rest R für einen Methyl- Ethyl- Propyl- 2-Ethyl- Hexyl-Cyclohexyl- Benzyl oder Phenylrest, wobei Methyl- und Ethylreste besonders bevorzugt sind. In der vorstehenden. allgemeinen Formel steht der Rest R' für einen aliphatischen, cycloaliphatischen, aromatischen oder aliphatisch aromatischen Kohlenwasserstoffrest mit 2 bis 20 Kohlenstoffatomen. Besonders bevorzugt werden als N-N-substituierte Diamine N,N-Dialkylaminoalkylamine, ganz besonders bevorzugt N,N-Dimethylaminoethylamin, N,N-Dimethylaminopropylamin, N,N-Diethylaminoethylamin oder N,N-Diethylaminopropylamin eingesetzt.

Es ist auch möglich, tertiäre, VOC-arme Amine zur Versalzung der Carboxylgruppen der Copolymere der Komponente I zu verwenden. Solche tertiären Amine sind beispielsweise alkoxilierte Amine, wie sie beispielsweise in US 2006/0089426 beschrieben sind, und von denen Bis[ω-hydrogenpoly[oxyethylen]oleylamin als repräsentative Verbindung genannt werden kann.

Als Komponente II der erfindungsgemäßen Mischung wird vorzugsweise ein bekannter Polyether verwendet, der zumindest aus 25 mol% Ethylenoxid, bevorzugt aus zu mindestens 50 mol% Ethylenoxid-Einheiten aufgebaut ist.

Solche Polyether werden vorzugsweise mit Hilfe von C₁ bis C₄ Monoalkoholen gestartet und weisen eine Hydroxidgruppe als Endgruppe auf.

Die Komponente II umfasst wenigstens einen wasserlöslichen Polyether, der aus zu mindestens 25 mol%, vorzugsweise zu wenigstens 50 mol% Ethylenoxid-Einheiten aufgebaut ist.

Neben den Ethylenoxid-Einheiten der erfindungsgemäß zum Einsatz kommenden Polyether und ggfs. weiteren Alkylenoxid-Einheiten können die Polyether auch auf Styroloxid-Einheiten und Glycidether-Einheiten basieren.

Neben den Ethylenoxid-Einheiten kann der als Komponente II eingesetzte wasserlösliche Polyether weitere Alkylenoxide-Elnheiten mit 3 bis 10 C-Atomen, vorzugsweise Propylenoxid und/oder Butylenoxid-Einheiten aufweisen. Darüber hinaus ist es auch möglich, dass neben den Ethylenoxid-Einheiten auch Einheiten von Styroloxid sowie aliphatischen oder aromatischn Glycidether mit 3 bis 20 C-Atomen, vorzugsweise Isopropylglycidether, n-Butylglycidether, Phenylglycidether und/oder 2-Ethylhexylglycidether, als Einheiten des zum Einsatz kommenden Polyethers vorliegen.

Darüber hinaus können die Polyether auch noch durch Estereinheiten kettenverlängert sein, die sich insbesondere von aliphatischen Lactonen mit 3 bis 10 C-Atomen, vorzugsweise vom Propionlacton, Valerianlacton und/oder ε-Caprolacton ableiten.

Das zahlengemittelte Molekulargewicht der erfindungsgemäß zum Einsatz kommenden Polyether beträgt vorzugsweise von 100 g/mol bis 2.000 g/mol.

Die Komponente III ist vorzugsweise wenigstens ein Triblockcopolymer, das durch Veresterung einer aliphatischen Dicarbonsäure mit einem wasserlöslichen Polyether oder einer Mischung von Polyethem, die mindestens einen wasserlöslichen Polyether enthält, erhalten worden ist. Der wasserlöslichen Polyether oder mindestens ein wasserlöslicher Polyether der Mischung von Polyethem entspricht vorzugsweise der Komponente (11).

Die aliphatische Dicarbonsäure besitzt vorzugsweise ein Molekulargewicht von 400 g/mol bis 1000 g/mol.

Als aliphatische, gesättigte oder ungesättigte Dicarbonsäure kann vorzugsweise eingesetzt werden:
- eine dimerisierte Fettsäure, die ggfs. gehärtet sein kann. Sie ist dem Fachmann auch als Dimersäure bekannt. Diese Dimersäuren können vorzugsweise durch Oligomerisation von ungesättigten Fettsäuren mit 12 bis 22 C-Atomen erhalten werden, vorzugsweise durch Oligomerisation von Ölsäure, Linolensäure und/oder Erucasäure. Dimerisierte Fettsäuren, d. h. Dimersäuren, mit einer Kohlenstoffketten bis zu 36 C-Atomen sind bevorzugt;
- ein Umsetzungsprodukt von cyclischen, aliphatischen ggfs. ungesättigten Dicarbonäureanhydriden mit aliphatischen Diolen oder Diaminen vorzugsweise im Verhältnis 2:1. Hierbei können vorzugsweise die cyclischen Dicarbonäureanhydriden wie Maleinsäureanhydrid oder Bernsteinsäureanhydrid eingesetzt werden.
   Die dazu zum Einsatz kommenden Diole sind vorzugsweise aliphatische, lineare, cyclische, oder verzweigte, Diole mit 2 bis 20 C-Atomen. Beispiele für solche Diole sind Ethylenglycol, 1,2-Propandiol, 1,3-Propandiol, 1,4-Butandiol, 1,2-Pentandiol und 1,6-Hexandiol.
   Darüberhinaus können die Diole auch durch Umsetzung mit aliphatischen Lactonen durch Oligo- oder Poly-Ester Bildung kettenverlängert sein. Diese dihydroxyterminierten Polyester können vorzugsweise durch Polymerisation einer oder mehrerer, gegebenenfalls alkylsubstituierter Lactone mit 3 bis 10 C-Atomen, wie zum Beispiel Propionlacton, Valerianlacton, Caprolacton mittels der oben beschrieben Diole als Startkomponenten, gemäß Angaben in EP-A-154 678 (US-A-4 647 647) erhalten werden.
   Die zur Umsetzung kommenden Diamine können vorzugsweise aliphatische, lineare, cyclische, oder verzweigte, Diamine mit 2 bis 20 C-Atomen und zwei primären Aminogruppen sein. Beispiele für solche Diamine sind Ethylendiamin, 1,2-Propandiamin, 1,3-Propandiamin, 1,6-Hexandiamin, Isophorondiamin und 3,3'-Dimethyl-4,4'-diaminodicyclohexylmethan.

Die Komponente (IV) ist ein Stempolymer, das durch Veresterung mindestens einer aliphatischen Multicarbonsäure, die drei bis zu fünf COOH-Gruppen aufweist, mit einem wasserlöslichen Polyether oder einer Mischung von Polyethern, die mindestens einen wasserlöslichen Polyether enthält, erhalten worden ist. Der wasserlösliche Polyether oder mindestens ein wasserlöslicher Polyether der Mischung von Polyethern entspricht vorzugsweise der Komponente (II).

Solche zum Einsatz kommenden aliphatischen Multicarbonsäuren weisen vorzugsweise ein Molekulargewicht von 500 g/mol bis 1500 g/mol auf. Wird eine Mischung von Multicarbonsäuren eingesetzt, so beträgt das zahlengemittelte Molekulargewicht ebenfalls von 500 g/mol bis 1500 g/mol.

Als aliphatische Multicarbonsäure mit drei bis fünf Carboxylgruppen werden vorzugsweise eingesetzt:
- eine trimerisierte Fettsäure, die ggfs. gehärtet sein kann. Sie ist dem Fachmann auch als Trimersäure bekannt. Trimerisierte Fettsäuren mit bis zu 54 C-Atomen sind bevorzugt.
- ein Umsetzungsprodukt von cyclischen, aliphatischen, ggfs. ungesättigten Dicarbonäureanhydriden mit aliphatischen Polyolen mit 3 bis 5 OH-Gruppen oder Polyaminen mit in der Summe 3 bis 5 primären und sekundären Aminogruppen im stöchiometrischen Verhältnis von einer Aminofunktion zu einer Anhydridfunktion. Hierbei können als cyclische Carbonäureanhydride vorzugsweise Maleinsäureanhydrid oder Bernsteinsäureanhydrid eingesetzt werden.
   Als Polyole können aliphatisch, lineare, cyclische, oder verzweigte Polyole mit 3 bis 20 C-Atomen und 3 bis 5 OH-Gruppen, wie zum Beispiel Glycerin eingesetzt werden.
   Darüberhinaus können die Polyole auch durch Umsetzung mit Lactonen durch Oligo- oder Polyester-Bildung kettenverlängert werden. Solche mit 3 bis 5 OH-Gruppen terminierte Polyester sind können durch Polymerisation einer oder mehrerer, gegebenenfalls alkylsubstituierter Lactone mit 3 bis 10 C-Atomen, wie zum Beispiel Propionlacton, Valerianlacton, Caprolacton mittels der vorstehend beschrieben Polyole als Startkomponenten und gemäß den Angaben in EP-A-154 678 (US-A-4 647 647), erhalten werden.
   Die bei der Umsetzung zum Einsatz kommenden Polyamine können vorzugsweise aliphatische, lineare, cyclische oder verzweigte Polyamine mit 3 bis 20 C-Atomen und mit in der Summe 3 bis 5 primären und sekundären Aminogruppen sein, wie zum Beispiel Dipropylentriamin.

Bei der Veresterungsreaktion zur Herstellung der Komponenten (III) und (IV) können die Dicarbonsäure und die Multicarbonsäure jeweils separat verestert werden. Sie können aber auch als Mischung zur Veresterung eingesetzt werden.

Bevorzugt wird zur Veresterung als Di- und Multicarbonsäure eine Dimer und Trimersäure eingesetzt, die je nach Hersteller und Reinheitsgrad als eine Mischung von monomerer Fettsäure, Dimersäure und Trimersäure mit unterschiedlichen Anteilen käuflich zu erwerben sind. Besonders die Trimersäure kann auch noch geringe Anteile an Oligomeren, die mehr als drei Carbonsäurefunktionen aufweisen, enthalten. Die nachstehend aufgeführten Dimersäuren enthalten üblicherweise kleiner < 8 Gew% monomere Fettsäuren und bis zu 25% Trimersäuren. Handelsübliche Prudukte an Dimersäure sind Empol^{®} 1008, Empol^{®} 1061, Empol^{®} 1062, Pripol^{®} 1006, Pripol^{®} 1017 und Pripol^{®} 1022.

Handelsübliche Produkte an Trimersäure sind Empol^{®} 1041 und Pripol^{®} 1040.

Ganz besonders bevorzugt werden als Di- bzw. Multicarbonsäuren zur Herstellung der Komponenten (III) und (IV) die aufgeführten Di- und Trimersäuren eingesetzt.

Die Veresterung der Di- und Multicarbonsäuren mit den Polyethern, vorzugsweise der Komponente II, wird mit dem Fachmann bekannten Verfahren durchgeführt. So kann die Reaktionsmischung in Gegenwart eines Veresterungskatalysators, der beispielsweise eine Sulfonsäure, Dibutylzinndilaurat oder ein tertiäres Amin ist, bei Reaktionstemperaturen bis zu 300°C bevorzugt bis zu 200°C durchgeführt werden. Das bei der Veresterung freiwerdende Wasser kann entweder azeotrop mit einem geeigneten Schleppmittel wie zum Beispiel Xylol, Benzol oder Tetrachlorkohlenstoff, oder durch Anlegen von Vakuum entfernt werden.

Die Veresterung wird vorzugsweise bis zu einem Umsetzungsgrad von mindestens 90% der Säurefunktionen der Di- und Multicarbonsäuren geführt.

Es kann bei der Veresterung vorzugsweise auch ein Überschuß an Polyether eingesetzt werden, der in der erfindungsgemäßen Mischung die Komponente (II) bildet.

In der erfindungsgemäßen Mischung kann aber auch als Komponente II ein weiterer Polyether, der unterschiedlich zu den in der Veresterung verwendeten Polyethern ist, vorliegen.

Das Triblockcopolymer der Komponente (III) und das Stempolymer der Komponente (IV) weisen ein wasserunlösliches Segment mit einem zahlengemittelten Molekulargewicht von 400 g/mol bis 1000 g/mol für Komponente III und von 500 g/mol bis 1500 g/mol für Komponente IV auf, wodurch die Pigmente in einer wässrigen Dispersion verbessert stabilisiert werden können. Wenn dieses Segment zu kurz oder zu wasserlöslich ist, so ist eine Pigmentdispersion nicht mehr stabil genug und die Pigmente flockulieren. Wenn das wasserunlösliche Segment zu lang ist, bilden die Komponenten (III) und (IV) Mizellen in Wasser. Allgemein zeigen Mizellen ein schlechteres Dispergierverhalten als die entsprechenden Unimere, so dass eine Mizellbildung nicht gewünscht ist.

Die erfindungsgemäßen Mischungen eignen sich als Netz- und Dispergiermittel für viele aus dem Stand der Technik bekannte Wasser basierende Einsatzzwecke. So können diese z. B. bei der Herstellung oder Verarbeitung von Lacken, Druckfarben, Tinten für inkjet-Verfahren wie für Tintenstrahldrucker, Papierstrich, Leder- und Textilfarben, Pasten, Pigmentkonzentraten, Keramiken, kosmetischen Zubereitungen eingesetzt werden, vorzugsweise immer dann, wenn Feststoffe wie Pigmente und/oder Füllstoffe vorhanden sind. Beispielsweise lassen sich die erfindungsgemäßen Mischungen bei der Herstellung von Industrielacken, Holz- und Möbellacken, Fahrzeuglacken, Schiffsfarben, Korrosionsschutzfarben, Can- und Coil-Coatings, Maler- und Bautenlacken verwenden, wobei ggf. übliche bekannte Bindemittel und/oder VOC-arme Lösungsmittel, Pigmente und gegebenenfalls Füllstoffe, die erfindungsgemäßen Polymermischungen und übliche Hilfsstoffe dazu gemischt werden.

Beispiele für übliche Bindemittel sind Harze auf Basis von Polyurethanen, Cellulosenitraten, Celluloseacetobutyraten, Alkyden, Melamin, Polyester, Chlorkautschuk, Epoxiden und Acrylaten.

Als Netz- und Dispergiermittel eigenen sich die erfindungsgemäßen VOC-armen Mischungen auch für die Herstellung von Beschichtungen auf Wasserbasis, wie kathodischen oder anodischen Elektrotauchlackierungen beispielsweise für Automobilkarossen. Weitere Beispiele für einen Einsatz als Dispergiermittel sind Putze, Silikatfarben, Dispersionsfarben, Wasserlacke auf Basis von wasserverdünnbaren Alkyden, Alkydemulsionen, Hybridsystemen, 2-Komponenten-Systemen, Polyurethan- und Acrylat-Dispersionen.

Die erfindungsgemäßen Mischungen eignen sich insbesondere auch zur Herstellung von Feststoffkonzentraten, vorzugsweise von Pigmentkonzentraten. Dazu werden diese in Wasser oder einer Mischung aus Wasser und VOC-armen organischen wassermischbaren Lösungsmitteln oder Weichmachern vorgelegt und die zu dispergierenden Feststoffe unter Rühren zugegeben. Zusätzlich können diese Konzentrate Bindemittel und/oder andere Hilfsstoffe enthalten.

Die Hilfsstoffe, die den Pigmentkonzentraten zugesetzt werden können, sind zum Beispiel
- Entschäumer, wie Mineralöl- und Silikonentschäumer;
- Rheologiesteuerungsmittel, wie Polyurethan-Verdicker, pyrogene Kieselsäuren, Polyamid- und Oligohamstoffverbindungen;
- Verlaufmittel,
- Antioxidantien,
- Biozide,
- organisch modifizierte Oligo- und Polysiloxane zur Oberflächenbenetzung.

Mit den erfindungsgemäßen Mischungen ist aber auch in vorteilhafter Weise möglich, stabile bindemittelfreie Pigmentkonzentrate herzustellen. Ebenso ist es möglich, mit den erfindungsgemäßen Mischungen fließfähige Pigmentkonzentrate aus Pigmentpresskuchen herzustellen. Hierbei wird dem Presskuchen, der noch Wasser enthalten kann, eine erfindungsgemäße Mischung zugemischt und die so erhaltene Mischung dispergiert. Solche Feststoffkonzentrate, vorzugsweise Pigmentkonzentrate, können dann in unterschiedliche Substrate wie z. B. Alkydharze, Polyesterharze, Acrylatharze, Polyurethanharze oder Epoxidharze eingearbeitet werden. Pigmente, die lösungsmittelfrei direkt in der erfindungsgemäße Mischungen dispergiert sind, eignen sich besonders zur Pigmentierung von thermoplastischen und duroplastischen Kunststoffformulierungen.

Die erfindungsgemäßen Polymermischungen können vorteilhaft auch bei der Herstellung von Tinten für "non impact"-Druckverfahren wie "thermal inkjet" und dem "Bubblejet-Verfahren" verwendet werden. Diese Tinten können beispielsweise wässrige Tintenformulierungen sein.

Die erfindungsgemäßen Mischungen können auch bei der Herstellung kosmetischer Zubereitungen wie zur Herstellung von Make-up, Puder, Lippenstiften, Haarfärbemitteln, Cremen, Nagellacken und Sonnenschutzpräparaten verwendet werden. Diese können in den üblichen Formulierungen wie als W/O- oder O/W-Emulsionen, Lösungen, Gele, Cremes, Lotionen oder Sprays, vorliegen. Die erfindungsgemäßen Mischungen können dabei in den zur Herstellung dieser Zubereitungen verwendeten Dispersionen bereits als Dispersionsmittel eingesetzt werden.

Ein weiterer Gegenstand der vorliegenden Erfindung ist auch die Verwendung der erfindungsgemäßen Mischungen als Netz- und Dispergiermittel. Vorzugsweise werden diese Netz- und Dispergiermittel für die vorstehend beschriebenen Einsatzzwecke verwendet.

Ein weiterer Einsatzzweck ist auch die Herstellung eines pigmentierten Überzuges auf einem Substrat, wobei der Pigmentlack auf das Substrat aufgebracht, und der aufgebrachte Pigmentlack eingebrannt oder ausgehärtet bzw. vernetzt wird.

Für die Einsatzzwecke der erfindungsgemäßen Mischungen können diese ggf. zusammen mit nach dem Stand der Technik üblichen Bindemitteln eingesetzt werden.

Eine erfindungsgemäße Verwendung besteht u. a. auch in der Herstellung dispergierbarer, pulverpartikel- und/oder faserpartikel-förmiger Feststoffe, insbesondere in der Herstellung von dispergierbaren Pigmenten, wobei die Partikel mit einer erfindungsgemäßen Mischung beschichtet sein können. Derartige Beschichtungen von organischen oder anorganischen Feststoffen werden in bekannter Weise, wie sie z. B. in EP-A-0 270 126 beschrieben, ausgeführt. Hierbei kann das VOC-arme Lösungs- oder Emulsionsmittel entweder entfernt werden oder im Gemisch unter Bildung einer Paste verbleiben. Solche Pasten sind gängige Handelsprodukte, die ggf. Bindemittel sowie weitere Hilfs- und Zusatzstoffe enthalten können.

Speziell bei Pigmenten kann die Modifizierung, d. h. Beschichtung der Pigmentoberfläche durch Zusatz der erfindungsgemäßen Mischungen während oder nach der Synthese der Pigmente erfolgen, d. h. durch deren Zugabe zur Pigmentsuspension oder während bzw. nach dem Pigmentfinish.

Die auf diese Weise vorbehandelten Pigmente zeichnen sich durch eine leichtere Einarbeitbarkeit und durch eine höhere Farbstärke gegenüber nicht Oberflächen behandelten Pigmenten aus.

Die erfindungsgemäßen Mischungen eignen sich als Netz- und Dispergierungsmittel für eine Vielzahl von Pigmenten wie Mono-, Di-, Tri- und Polyazopigmente, Oxazin-, Dioxazin-, Thiazin-Pigmente, Diketo-pyrrolo-pyrrole, Phthalocyanine, Ultramarin und andere Metallkomplex-Pigmente, indigoide Pigmente, Diphenylmethan-, Triarylmethan-, Xanthen-, Acridin-, Chinacridon-, Methin-Pigmente, Anthrachinon-, Pyranthron-, Perylen- und andere polycyclische Carbonyl-Pigmente. Weitere Beispiele für erfindungsgemäß dispergierbare organische Pigmente finden sich in der Monographie: W. Herbst, K. Hunger "Industrial Organic Pigments", 1997 (Verlag: Wiley-VCH, ISBN: 3-527-28836-8). Beispiele für erfindungsgemäß dispergierbare, anorganische Pigmente sind Pigmente auf Basis von Ruß, Graphit, Zink, Titandioxid, Zinkoxid, Zinksulfid, Zinkphosphat, Bariumsulfat, Lithophone, Eisenoxid, Ultramarin, Manganphosphat, Cobaltaluminat, Cobaltstannat, Cobaltzinkat, Antimonoxid, Antimonsulfid, Chromoxid, Zinkchromat, gemischten Metalloxiden auf Basis von Nickel, Bismut, Vanadlum, Molybdän, Cadmium, Titan, Zink, Mangan, Cobalt, Eisen, Chrom, Antimon, Magnesium, Aluminium (beispielsweise Nickeltitangelb, Bismutvanadat-molybdatgelb oder Chromtitangelb). Weitere Beispiele sind in der Monographie: G. Buxbaum "Industrial Inorganic Pigments", 1998 (Verlag: Wiley-VCH, ISBN: 3-527-28878-3) genannt. Anorganische Pigmente können auch magnetische Pigmente auf Basis von Reineisen, Eisenoxiden und Chromoxiden oder Mischoxiden, Metalleffektpigmente aus Aluminium, Zink, Kupfer oder Messing sowie Perlglanzpigmente, fluoreszierende und phosphoreszierende Leuchtpigmente sein. Mit Hilfe der erfindungsgemäßen Polymermischungen können auch nanoskalige, organische oder anorganische Feststoffe mit Teilchengrößen unterhalb von 100 nm, wie bestimmte Rußtypen oder Partikel, die aus einem Metall-, oder Halbmetalloxid bzw. -hydroxid bestehen, sowie Partikel dispergiert werden, die aus gemischten Metall- und/oder Halbmetalloxiden bzw. -hydroxiden bestehen. Hierfür geeignete Oxide sind Oxide und/oder Oxidhydroxide des Aluminiums, Siliziums, Zinks, Titans, die zur Herstellung solch extrem feinteiligen Feststoffe eingesetzt werden können. Der Herstellprozess dieser oxidischen bzw. hydroxidischen bzw. oxidhydroxidischen Teilchen kann über verschiedene Verfahren z. B. Ionenaustauschprozesse, Plasmaprozesse, Sol-Gel-Verfahren, Ausfällung, Zerkleinerung (z. B. durch Vermahlung) oder Flammhydrolyse usw. erfolgen. Bei diesen nanoskaligen Feststoffen kann es sich auch um sogenannte Hyberid-Partikeln handeln, die aus einem anorganischen Kern und einer organischen Hülle oder umgekehrt aufgebaut sind.

Erfindungsgemäß dispergierbare, pulver- oder faser-förmige Füllstoffe sind u. a. solche, die aus pulver- oder faser-förmigen Teilchen von Aluminiumoxid, Aluminiumhydroxid, Siliziumdioxid, Kieselgur, Kieselerde, Quarz, Kieselgel, Talkum, Kaolin, Glimmer, Perlite, Feldspat, Schiefermehl, Calciumsulfat, Bariumsulfat, Calciumcarbonat, Calcit, Dolomit, Glas oder Kohlenstoff aufgebaut sind. Weitere Beispiele für dispergierbare Pigmente oder Füllstoffe finden sich auch in der EP-A-0 270 126. Auch Mattierungsmittel wie z. B. Kieselsäuren lassen sich ebenfalls mit den erfindungsgemäßen Mischungen hervorragend dispergieren und stabilisieren.

Ein weiterer Gegenstand der vorliegenden Erfindung sind daher auch Lacke und Pasten enthaltend wenigstens eine erfindungsgemäße Mischung und wenigstens ein Pigment, Wasser und ggf. ein VOC-armes, organisches Trägermedium, sowie gegebenenfalls Bindemittel und übliche Hilfsstoffe.

Ein weiterer Gegenstand der vorliegenden Erfindung sind daher auch die vorstehend genannten Pigmente beschichtet mit wenigstens einer erfindungsgemäßen Mischung.

### Beispiele:

### I Herstellung der Copolymere mit Carboxylgruppen

### Polymer 1: alternierendes SMA-Harz mit einem Styrol zu MSA-Verhältnis von 2:1

27,3 g Methoxypropylacetat, 4,2 g 2,4-Diphenyl-4-methyl-1-penten und 3,3 g Styrol werden auf 140°C erhitzt. Nach Erreichen der Reaktionstemperatur werden 14,4 g Maleinsäureanhydrid und 2,3 g AMBN in 21,7 g Methoxypropylacetat angelöst, in 100 min hinzudosiert und 26,8 g Styrol in 85 min hinzudosiert.
Nach 1 h Nachreaktionszeit wird die Polymerlösung auf Raumtemperatur abgekühlt. Restgehalt an 2,4-Diphenyl-4-methyl-1-penten: 0,1%
Mₙ: 2775g/mol

### Polymer 2: alternierendes SMA-Harz mit einem Styrol zu MSA-Verhältnis von 1:1

11,3 g Methoxypropylacetat und 5,8 g 2,4-Diphenyl-4-methyl-1-penten werden auf 140°C erhitzt. Nach Erreichen der Reaktionstemperatur werden 20,4 g Maleinsäureanhydrid und 2,8 g AMBN in 38,1 g Methoxypropylacetat angelöst, in 100 min hinzudosiert und 21,6g Styrol in 100 min hinzudosiert.
Nach 1 h Nachreaktionszeit wird die Polymerlösung auf Raumtemperatur abgekühlt. Restgehalt an 2,4-Diphenyl-4-methyl-1-penten: 0,1%
Mₙ: 1886g/mol

### Polymer 3: Diblockcopolymer

27,3 g Methoxypropylacetat, 4,2 g 2,4-Diphenyl-4-methyl-1-penten werden auf 140°C erhitzt. Nach Erreichen der Reaktionstemperatur werden 14,4 g Maleinsäureanhydrid und 2,3 g AMBN in 21,7 g Methoxyproplacetat angelöst in 100 min hinzudosiert und 26,8 g Styrol in 85 min hinzudosiert. Nach 15 min Nachreaktionszeit wird 1 g AMBN in 10 g Methoxypropylenacetat angelöst und 10 g Styrol in 100 min hinzudosiert.
Nach 1 h Nachreaktionszeit wird die Polymerlösung auf Raumtemperatur abgekühlt. Restgehalt an 2,4-Diphenyl-4-methyl-1-penten: 0,08%
Mₙ: 3156 g/mol

### Polymer 4: Diblockcopolymer

22,8 g Methoxypropylacetat, 13,8 g BlocBuilder und 31,2g Styrol werden auf 120°C erhitzt. Nach Erreichen der Reaktionstemperatur werden 24,7 g Acrylsäure in 7,6 g Methoxyproplacetat angelöst in 100 min hinzudosiert. Nach 2 h Nachreaktionszeit wird die Polymerlösung auf Raumtemperatur abgekühlt.
Mₙ: 2835 g/mol

Die Molekulargewichte wurden mittels Gelpermeationschromatrographie mit THF als Laufmittel und Polystyrol als Standard gemessen.

### II Versalzte Copolymere

### Polymerlösung 1

100 g einer Lösung des Polymers 1 in Methoxypropylacetat werden mit 10,7g NaOH und 91,05 g Wasser versetzt und auf 100°C erhitzt, wobei das Methoxypropylacetat als Azeotrop abdestilliert wird.
Die Mischung wird mit Wasser auf einen Festkörper von 40 Gew% verdünnt.

### Polymerlösung 2

100 g einer Lösung des Polymers 2 in Methoxypropylacetat werden mit 23,6g KOH und 110,4g Wasser versetzt und auf 100°C erhitzt, wobei das Methoxypropylacetat als Azeotrop abdestilliert wird.
Die Mischung wird mit Wasser auf einen Festkörper von 40 Gew % verdünnt.

### Polymerlösung 3

100 g einer Lösung des Polymers 2 in Methoxypropylacetat werden mit 21,4g Dimethylaminopropylamin und 107,1g Wasser versetzt und auf 100°C erhitzt, wobei das Methoxypropylacetat als Azeotrop abdestilliert wird.
Die Mischung wird mit Wasser auf einen Festkörper von 40 Gew % verdünnt.

### Polymerlösung 4

12,2 g KOH werden in 60 g Wasser gelöst und anschließend werden 27,78 g SMA 1000-Harz bei 80°C gelöst.

### Polymerlösung 5

100 g einer Lösung des Polymers 4 in Methoxypropylacetat werden mit 21,5 g KOH und 122,3 g Wasser versetzt und auf 100°C erhitzt, wobei das Methoxypropylacetat als Azeotrop abdestilliert wird.
Die Mischung wird mit Wasser auf einen Festkörper von 40 Gew% verdünnt.

### Polymerlösung 6

16 g KOH werden in 250 g Wasser gelöst und anschließend werden 50 g SMA 1000-Harz bei 80°C gelöst. Anschließend werden 150 g eines alkoxilierten, tertiären Diamins (Beispiel 9 aus US 2006/0089426) hinzugefügt.
Die Mischung wird mit Wasser auf einen Festkörper von 40 Gew% verdünnt.

### III Veresterungsprodukte

### Ester 1:

10,04 g Pripol 1022 werden mit 29,92 g Pluriol A 750 E und 0,1 g para-Toluolsulfonsäure vorgelegt und bei 210°C zur Reaktion gebracht, wobei das entstehende Wasser abgetrennt wird. Die Reaktion ist beendet, wenn 90% der Carboxylgruppen verestert sind. Anschließend wird der Festkörper mit Wasser auf 40 Gew% eingestellt.

### Ester 2:

24,35 g Pripol 1022 werden mit 202,7 g Pluriol A2300PE und 0,15 g para-Toluolsulfonsäure vorgelegt und bei 210°C zur Reaktion gebracht, wobei das entstehende Wasser abgetrennt wird. Die Reaktion ist beendet, wenn 90% der Carboxylgruppen verestert sind. Anschließend wird der Festkörper mit Wasser auf 40 Gew% eingestellt.

### Ester 3 (Vergleichsbeispiel):

10,04 g Pripol 1022 werden mit 14,96 g Pluriol A 750 E und 0,06 g para-Toluolsulfonsäure vorgelegt und bei 210°C zur Reaktion gebracht, wobei das entstehende Wasser abgetrennt wird. Die Reaktion ist beendet, wenn nur 50% der Carboxylgruppen verestert sind. Anschließend wird der Festkörper mit Wasser auf 40 Gew% eingestellt.

### Ester 4 (Vergleichsbeispiel):

8,72 g Tallölfettsäure werden mit 26,1 g Pluriol A 750 E und 0,06 g para-Toluolsulfonsäure vorgelegt und bei 210°C zur Reaktion gebracht, wobei das entstehende Wasser abgetrennt wird. Die Reaktion ist beendet, wenn 90% der Carboxylgruppen verestert sind. Anschließend wird der Festkörper mit Wasser auf 40 Gew% eingestellt.

### IV Mischung aus versalzten Copolymeren (Produkte II) und aus Veresterungsprodukten (Produkte III)

### Netz und Dispergiermittel 1 (erfindungsgemäß) (N+D1)

50g von Polymerlösung 4 werden mit 50g von Ester 1 gemischt und homogenisiert.

### Netz und Dispergiermittel 2 (Vergleichsbeispiel) (N+D2)

50g von Polymerlösung 4 werden mit 50g von Ester 3 gemischt und homogenisiert.

### Netz und Dispergiermittel 3 (Vergleichsbeispiel) (N+D3)

50g von Polymerlösung 4 werden mit 50g von Ester 4 gemischt und homogenisiert.

| | |
|---|---|
| BlocBuilder: | Polymerisationsinitiator, Hersteller Arkema |
| SMA 1000-Harz: | Styrol/Maleinsäureanhydrid-Copolymer; Hersteller: Cray Valley |
| Pripol 1022: | Dimersäure (1-3% Monosäure, 74-85% Dimersäure, 15-23% Trimersäure); Hersteller: UniQuema |
| Pluriol A750E: | Methanol gestarteter Polyetherglykol; Hersteller: BASF |
| Pluriol A2300PE: | 1-Butanol gestarteter EO- PO Polyether; Hersteller: BASF |

### V Anwendungstests

### 5.1 Formulierungen

### 5.1.1 Wässrige Pigmentkonzentrate:

| | Tronox CR 826 | Monarch 120 | HeliogenBlau L7101F |
|---|---|---|---|
| Wasser N+D-Mittel 1,2 bzw. 3 bzw. Ester 1 bzw. SMA- | 20,15 | 58,9 | 37,65 |
| Lsg. 4 | 3,75 | 20 | 26,25 |
| BYK®-024 | 1,0 | 1,0 | 1,0 |
| Parmetol A26 | 0,1 | 0,1 | 0,1 |
| Pigment | 75 | 20 | 35,0 |
| Gew% | 100,00 | 100,00 | 100,00 |

| | | | |
|---|---|---|---|
| N+D-Mittel = Netz- und Dispergierungsmittel | | | |

Das Pigmentkonzentrat, enthaltend das Tronoxpigment, wird mit einem Dissolver bei 10 m/s Rührgeschwindigkeit und einer Dispergierzeit 29 min hergestellt.

Die Dispergierung der beiden anderen Pigmente erfolgt über 120 min mit Hilfe eines Scandex Rüttlers und Glasperlen.

### 5.1.2 Pigmentierte Lacke:

| **Klarlack** | |
|---|---|
| Mowilith LDM 7416 | 71,30 |
| Wasser | 13,30 |
| AMP 90 | 0,20 |
| Propylenglykol | 1,50 |
| Texanol | 2,00 |
| Wasser | 3,60 |
| BYK-028 | 0,50 |
| Tafigel PUR 40 | 0,30 |
| Parmetol A 26 | 0,20 |

| Dissolver 5 min. 3m/s | |
|---|---|
| BY-348 | 0,50 |
| Wasser | 6,60 |
| **Summe Gew%** | **100,00** |

### Auflackungen:

Tronox (als Pigment):

| | |
|---|---|
| Pigmentkonzentrat | 12 g |
| Klarlack | 30 g |
| | 42 g |

Heliogenblau bzw. Monarch (als Pigment):

| | |
|---|---|
| Pigmentkonzentrat | 3 g |
| Klarlack | 30 g |
| | 33 g |

Das Pigmentkonzentrat wird mit dem Lack 5 min gerüttelt.
Tronox CR 826: Titandioxidpigment, Hersteller: Tronox
Heliogenblau L71 01 F: Phthalocyaninpigment, Hersteller: BASF
Monarch 120: Russpigment, Hersteller: Cabot
BYK®-024: Entschäumer, Hersteller: Byk Chemie GmbH
Parmetol A26: Konservierungsmittel, Hersteller: S&M-Chemie
Mowilith LDM7416: Styrol/Acrylat-Copolymer-Dispersion, Hersteller: Celanese
AMP90: 2-Amino-2-Methylpropanol, Hersteller: Dow
Tafigel PUR 40: Assoziatiwerdicker, Hersteller: Münzing-Chemie
BYK®-028:, Entschäumer, Hersteller: Byk Chemie GmbH
BYK®348: Substratbenetzmittel, Hersteller: Byk Chemie GmbH
Texanol: Koaleszenzmittel, Hersteller: Eastman
Propylenglycol: Lösemittel, Hersteller Dow

### 5.2 Testergebnisse

### 5.2.1 Wässrige Pigmentkonzentrate

| Pigment | N+D-Mittel | Beurteilung der Pigmentkonzentrate | | |
|---|---|---|---|---|
| | | Viskosität | Komfeinheit | Lagerung 1 Tag, RT |
| Tronox | Ester 1 (Vergleich) | nicht dispergierbar | | - |
| | SMA-Lsg 4 (Vergleich) | fließfähig | 15 µm | 1 |
| | N+D 1 | fließfähig | 15 µm | 1 |
| | N+D 2 (Vergleich) | fließfähig | 15 µm | 2 |
| | N+D 3 (Vergleich) | fließfähig | 15 µm | 2 |
| Heliogenblau | Ester 1 | fließfähig | 15 µm | 1 |
| | SMA-Lsg 4 | hochviskos | 60 -100 µm | 3 |
| | N+D 1 | fließfähig | 15 µm | 1 |
| | N+D 2 | fließfähig | 25 µm | 1 |
| | N+D 3 | fließfähig | 10 µm | 1 |
| Monarch | Ester 1 | fließfähig | 10 µm | 1 |
| | SMA-Lsg 4 | schaumig | >100 µm | 2 |
| | N+D 1 | fließfähig | 10 µm | 1 |
| | N+D2 | fließfähig | 10 µm | 1 |
| | N+D3 | fließfähig | 10 µm | 1 |

| | | | | |
|---|---|---|---|---|
| 1: kein Absetzen der Pigmente, kein bis ein geringfügiger Anstieg der Viskosität 2: Absetzen der Pigmente 3: kein Absetzen der Pigmente, starker Anstieg der Viskosität | | | | |

Die Messung der Kornfeinheit erfolgte mit einem 100 µm-Grindometer im nassen Lack, da die wässrigen Pigmentkonzentrate in keinem durchgängigen Film wegen Benetzungsstörungen auf dem Grindometer appliziert werden können.

### 5.2.2 Auflackungen

| Pigment | N+D-Mittel | Glanz (20°) | Glanz (60°) | Stippen |
|---|---|---|---|---|
| Tronox | Ester 1 | keine Auflackung möglich | | |
| | SMA-Lsg 4 | 19,7 | 52,2 | keine |
| | N+D 1 | 15,8 | 55,8 | keine |
| | N+D 2 | 13,4 | 54,7 | keine |
| | N+D 3 | 18,4 | 58,7 | keine |
| Heliogenblau | Ester 1 | 66,1 | 80 | keine |
| | SMA-Lsg 4 | 58,3 | 77,5 | viele |
| | N+D 1 | 69,9 | 81,6 | keine |
| | N+D2 | 65,3 | 79,4 | keine |
| | N+D3 | 67,3 | 80,0 | keine |
| Monarch | Ester 1 | 65,7 | 80,6 | keine |
| | SMA-Lsg 4 | 12,0 | 42,6 | viele |
| | N+D 1 | 66,5 | 81,4 | keine |
| | N+D2 | 65,3 | 80,7 | keine |
| | N+D 3 | 65,1 | 80,6 | keine |

Die Glanzmessungen erfolgten mit einem color guide sphere von BYK-Gardner.

Die Ergebnisse zeigen, dass mit den Einzelkomponenten der Mischung, den nichtionischen Polyether-haltigen Komponenten (Ester 1) einerseits und dem versalzten Copolymer (SMA-Lösung 4) andererseits, nicht mit allen ausgewählten Pigmenten Pigmentkonzentrate und Auflackungen mit einer hinreichenden Qualität erhalten werden. Aus mit Ester 1 und dem anorganischen Pigment Tronox kann kein Pigmentkonzentrat erzeugt werden.

Das versalzte saure Copolymer ist für das anorganische Pigment Tronox geeignet, zeigt aber bei der Verwendung der beiden organischen Pigmente Schwächen (hochviskose oder schaumige Pigmentkonzentrat, in denen die Pigmente teilweise noch als Agglomerate vorliegen und Auflackungen, die Stippen enthalten).

Mit der erfindungsgemäße Mischung (Netz- und Dispergiermittel 1) können sowohl mit dem anorganischen Pigment als auch mit den beiden organischen Pigmenten fließfähige und lagerstabile Pigmentkonzentrate, die sich zur Formulierung von stippenfreien Lacken eignen, hergestellt werden.

Die Tronoxpigmentkonzentrate, die die Vergleichsbeispiele Netz- und Dispergiermittel 2 und 3 enthalten, zeigen eine geringere Lagerstabilität als die erfindungsgemäße Mischung.

## Patentansprüche

1. Eine an flüchtigen organischen Verbindungen (VOC) arme Mischung, deren Anteil an leicht flüchtigen, organischen Lösungsmittel nicht die nach ISO 11890-2 bzw. DIN 55649 festgelegten Grenzwerte überschreiten darf, aus
I 10 bis 80 Gew-% eines zumindestens zu 50 % versalzten Copolymeren aus wenigstens einem ethylenisch ungesättigten, phenylgruppenhaltigen Monomeren und wenigstens einer α,β-ungesättigten Monocarbonsäure und/oder wenigstens einer α,β-ungesättigten Dicarbonsäure und ggfs. deren Derivaten,
II 1 bis 30 Gew-% wenigstens eines wasserlöslichen, eine endständige OH-Gruppe aufweisenden, linearen Polyethers, der zu mindestens aus 25 mol-% aus Ethylenoxid aufgebaut ist,
III 5 bis 80 Gew-% wenigstens eines Veresterungsprodukts aus einer aliphatischen Dicarbonsäure und aus wenigstens einem der wasserlöslichen Polyether der Komponente II mit vorzugsweise einem zahlgemittelten Molekulargewicht von ≤ 2000 g/mol oder einer Mischung von Polyethern, die mindestens einen wasserlöslichen Polyether der Komponente II mit vorzugsweise einem zahlengemittelten Molekulargewicht von 2000 g/mol aufweist.
und
IV 3 bis 80 Gew-% mindestens eines Sternpolymeren erhältlich durch Veresterung von wenigstens einer drei bis fünf Carboxylgruppen aufweisenden, aliphatischen Carbonsäure mit wenigstens einem der wasserlöslichen Polyether der Komponente II mit vorzugsweise einem zahlgemittelten Molekulargewicht von ≤ 2000 g/mol oder einer Mischung von Polyethern, die mindestens einen wasserlöslichen Polyether der Komponente II mit vorzugsweise einem zahlengemittelten Molekulargewicht von ≤ 2000 g/mol aufweist,
wobei die Summe der Gew% der Komponenten I bis IV immer 100 Gew% ergeben muss und die Gew% der Komponenten I-IV jeweils auf das Gesamtgewicht der Komponenten I - IV bezogen sind.

2. Eine Mischung gemäß Anspruch 1, **dadurch gekennzeichnet, dass** sie 30 bis 70 Gew-% der Komponente I,
2 bis 10 Gew-% der Komponente II,
10 bis 50 Gew-% der Komponente III und
5 bis 20 Gew-% der Komponente IV
enthält,
wobei die Summer der Gew-% der Komponente I bis IV immer 100 Gew% ergeben muss und die Gew% der Komponenten I bis IV jeweils auf das Gesamtgewicht der Komponente I bis IV bezogen sind.

3. Eine Mischung gemäß Anspruch 1 oder 2, **dadurch gekennzeichnet, dass** die Komponente I zumindest zu 75 % versalzt ist und vorzugsweise als Alkalimetall-, Erdalkalimetall- oder Ammonium- Salz, besonders bevorzugt als Natrium- oder KaliumSalz, vorliegt.

4. Eine Mischung gemäß einem der Ansprüche 1 bis 3, **dadurch gekennzeichnet, dass** das versalzte Copolymer der Komponente I durch Polymerisation von wenigstens einem ethylenisch ungesättigten, phenylgruppenhaltigen Monomeren mit vorzugsweise 8 bis 20 C-Atomen, wobei die Phenylreste ggfs. wenigstens einfach substituiert sein können, und von wenigstens einer α,β-ungesättigten Monocarbonsäure mit vorzugsweise 3 bis 8 Kohlenstoffatomen und/oder von wenigstens einer α,β-ungesättigten Dicarbonsäure mit vorzugsweise 4 bis 10 Kohlenstoffatomen und/oder deren cyclischen Anhydriden und ggfs. deren jeweiligen Derivaten, vorzugsweise Estern und Amiden, hergestellt worden ist.

5. Eine Mischung gemäß Anspruch 4, **dadurch gekennzeichnet, dass** als ethylenisch ungesättigtes, phenylgruppenhaltiges Monomeres wenigstens ein Monomeres ausgewählt aus der Gruppe umfassend Styrol, Benzyl(meth)acrylat und Phenylacrylat, wobei die Phenylreste ggfs. 1- bis 4-fach substituiert sein können, vorzugsweise ggfs. substituiertes Styrol, eingesetzt worden ist, und
als α,β-ungesättigte Monocarbonsäure Acrylsäure oder Methacrylsäure und als α,β-ungesättigte Dicarbonsäure und/oder deren cyclisches Anhydrid wenigstens eine Dicarbonsäure ausgewählt aus der Gruppe umfassend Maleinsäure, Fumarsäure und Itakonsäure und/oder Maleinsäureanhydrid, und ggfs, als deren jeweiliges Derivat, vorzugsweise Ester oder Amid, eingesetzt worden ist.

6. Eine Mischung gemäß einem der Ansprüche 1 bis 5, **dadurch gekennzeichnet, dass** die Komponente I ein versalztes Styrol/Maleinsäureanhydridcopolymeres mit einem Styrol zu Maleinsäureanhydrid-Verhältnis von 1:1 bis 8:1, vorzugsweise von 1:1 bis 2:1 ist.

7. Eine Mischung gemäß einem der Ansprüche 1 bis 6, **dadurch gekennzeichnet, dass** die Komponente II wenigstens ein wasserlöslicher Polyether ist, der zumindest zu 50 mol% aus Ethylenoxid aufgebaut ist.

8. Eine Mischung gemäß einem der Ansprüche 1 bis 7, **dadurch gekennzeichnet, dass** die Komponente II wenigstens einen wasserlöslichen Polyether ist, der aus Einheiten des Ethylenoxids und aus Einheiten wenigstens eines weiteren Alkylenoxids, vorzugsweise Propylenoxid, Butylenoxid und/oder Styroloxid oder
aus Ethylenoxid-Einheiten und aus Einheiten wenigstens eines aliphatischen oder aromatischen Glycidethers mit 3 bis 20 C-Atomen, vorzugsweise Isopropylglycidether, n-Butylglycidether, Phenylglycidether oder 2-Ethylhexylpolyglycidether, und/oder aus Einheiten wenigstens eines aliphatischen Lactons mit 3 bis 10 C-Atomen, vorzugsweise Propionlacton, Valerianlacton und/oder ε-Caprolacton aufgebaut ist.

9. Eine Mischung gemäß einem der Ansprüche 1 bis 8, **dadurch gekennzeichnet, dass** die Komponente III das Veresterungsprodukt aus wenigstens einem wasserlöslichen Polyether der Komponente II oder aus einer Mischung von Polyethern, die mindestens einen wasserlöslichen Polyether der Komponente II enthält, und einer aliphatischen, ggfs. gesättigten Dimersäure ist, die durch Oligomerisation von ungesättigten Fettsäuren mit 12 bis 22 C-Atomen erhältlich ist.

10. Eine Mischung gemäß einem der Ansprüche 1 bis 9, **dadurch gekennzeichnet, dass** die Komponente III das Veresterungsprodukt aus wenigstens einem wasserlöslichen Polyether der Komponente II oder einer Mischung von Polyethern, die mindestens einen wasserlöslichen Polyether der Komponente II enthält, und
einer ggfs. teilweise veresterten, aliphatischen Dicarbonsäure ist, die durch Umsetzung eines ggfs. ungesättigten, aliphatischen Dicarbonsäureanhydrids, vorzugsweise Maleinsäure- oder Bernsteinsäureanhydrids, mit wenigstens einem aliphatischen Diol mit C₂ bis C₂₀, das ggfs. durch Umsetzung mit einem dihydroxy-terminierten, aliphatischem Polyester basierend auf wenigstens einem aliphatischen Lacton mit 3 bis 10 C-Atomen kettenverlängert sein kann, erhältlich ist, oder
einer ggfs. teilweise amidierten, aliphatischen Dicarbonsäure ist, die durch Umsetzung eines ggfs. ungesättigten, aliphatischen Dicarbonsäureanhydrids, vorzugsweise Maleinsäure- oder Bemsteinsäureanhydrids, mit wenigstens einem linearen oder verzweigten, aliphatischen oder cycloaliphatisches, zwei primäre Aminogruppen aufweisenden Diamin mit 2 bis 20 C-Atomen erhältlich ist.

11. Eine Mischung gemäß einem der Ansprüche 1 bis 10, **dadurch gekennzeichnet, dass** die Komponente IV das Veresterungsprodukt aus wenigstens einem der wasserlöslichen Polyether der Komponente II oder einer Mischung von Polyethern, die mindestens einen wasserlöslichen Polyether der Komponente II enthält, und einer ggfs, gesättigten Trimersäure ist, die durch Oligomerisation einer ungesättigten Fettsäure mit 12 bis 22 C-Atomen erhältlich ist.

12. Eine Mischung gemäß einem der Ansprüche 1 bis 11, **dadurch gekennzeichnet, dass** die Komponente IV das Veresterungsprodukt von wenistens einem wasserlöslichen Polyether der Komponente II oder einer Mischung von Polyethern, die mindestens einen wasserlöslichen Polyether der Komponente II enthält, und
wenigstens einer aliphatischen, ggfs. teilveresterten 3 bis 5 Carboxylgruppen aufweisenden Multicarbonsäure ist, die durch Umsetzung eines ggfs. ungesättigten, aliphatischen Dicarbonsäureanhydrids, vorzugsweise Maleinsäure- oder Bemsteinsäureanhydrids, mit wenigstens einem aliphatischen Polyol mit 3 bis 5 OH-Gruppen, das ggfs. durch Umsetzung mit einem auf aliphatischen Lactonen mit 3 bis 10 C-Atomen basierenden Polyester kettenveriängert ist, in einem stöchiometischen Verhältnis der Anhydridfunktion zur Hydroxyl-Funktion von 1:1 erhältlich ist, oder
einer aliphatischen, ggfs. amidierten 3 bis 5 Carboxylgruppen aufweisenden Multicarbonsäure ist, die durch Umsetzung eines ggfs. ungesättigten, aliphatischen Dicarbonsäureanhydrids, vorzugsweise Maleinsäure- oder Bernsteinsäureanhydrids, mit aliphatischen Polyaminen mit in der Summe 3 bis 5 primären und/oder sekundären Aminogruppen in einem stöchiometischen Verhältnis der Anhydridfunktion zur Aminofunktion von 1:1 erhältlich ist.

13. Eine Mischung gemäß einem der Ansprüche 1 bis 11, **dadurch gekennzeichnet, dass** die Komponente II und IV das Veresterungsprodukt einer Mischung aus Dimer- und Trimersäure, die durch Oligomerisation von ungesättigten Fettsäuren mit 12 bis 22 C-Atomen erhältlich ist, mit wenigstens einem der wasserlöslichen Polyether der Komponente II oder einer Mischung von Polyethern ist, die mindestens einen wasserlöslichen Polyether der Komponente II enthält, wobei vorzugsweise das Verhältnis von Dimersäure zu Trimersäure in der eingesetzten Mischung 10:90 bis 90:10 beträgt und vorzugsweise die Dimersäure bis zu 36 C-Atomen und die Trimersäure bis zu 54 C-Atomen aufweist.

14. Verwendung einer Mischung gemäß einem der Ansprüche 1 bis 13 als VOC-armes Netz- und Dispergiermittel, dessen Anteil an leicht flüchtigen, organischen Lösungsmittel nicht die nach ISO 11890-2 bzw. DIN 55649 festgelegten Grenzwerte überschreiten darf.

15. Verwendung gemäß Anspruch 14 zur Dispergierung von Feststoffen, vorzugsweise zur Dispergierung von Pigmenten, oder zur Herstellung eines VOC-armes, wässrigen Pigmentkonzentrats, dessen Anteil an leicht flüchtigen, organischen Lösungsmittel nicht die nach ISO 11890-2 bzw. DIN 55649 festgelegten Grenzwerte überschreiten darf.

16. Verwendung eines Pigmentkonzentrats gemäß Anspruch 15 zur Herstellung von Überzugsmaterialien, vorzugsweise Lacken.

17. Wässriges, VOC-armes Pigmentkonzentrat enthaltend als Netz- und Dispergiermittel eine Mischung gemäß einem der Ansprüche 1 bis 13, dessen Anteil an leicht flüchtigen, organischen Lösungsmittel nicht die nach ISO 11890-2 bzw. DIN 55649 festgelegten Grenzwerte überschreiten darf.

18. Ein Lack oder eine Paste enthaltend wenigstens ein Pigment, eine Mischung gemäß einem der Ansprüche 1 bis 13, ein wässriges Dispersionsmedium und ggfs. ein VOC-armes, organisches Trägermedium, dessen Anteil an leicht flüchtigen, organischen Lösungsmittel nicht die nach ISO 11890-2 bzw. DIN 55649 festgelegten Grenzwerte überschreiten darf, und ggfs. weitere Hilfsstoffe.

19. Ein dispergierbarer, multipartikulärer und/oder faserförmiger Feststoff, vorzugsweise ein Pigment, beschichtet mit einer Mischung gemäß einem der Ansprüche 1 bis 13.

20. Verwendung einer Mischung gemäß einem der Ansprüche 1 bis 13 zur Herstellung einer Tinte, vorzugsweise einer wässrigen Tinte für das .non impact" Druckverfahren, oder als Netz- und Dispergiermittel in kosmetischen Zubereitungen, vorzugsweise W/O- oder O/W-Emulsionen, Lösungen, Gele, Cremes, Lotionen oder Sprays.

## Claims

1. A mixture low in volatile organic compounds (VOC) content, where the fraction of volatile organic solvents must not exceed the limits defined according to ISO 11890-2 or DIN 55649, and composed of
I 10% to 80% by weight of an at least 50% salified copolymer of at least one ethylenically unsaturated, phenyl-group-containing monomer and at least one α,β-unsaturated monocarboxylic acid and/or at least one α,β-unsaturated dicarboxylic acid, and optionally derivatives thereof,
II 1% to 30% by weight of at least one water-soluble, linear polyether which contains a terminal OH end group and is synthesized to an extent of at least 25 mol% from ethylene oxide,
III 5% to 80% by weight of at least one esterification product of an aliphatic dicarboxylic acid and of at least one of the water-soluble polyethers of component II having preferably a number-average molecular weight of ≤ 2000 g/mol or of a mixture of polyethers which comprises at least one water-soluble polyether of component II having preferably a number-average molecular weight of ≤ 2000 g/mol,
and
IV 3% to 80% by weight of at least one star polymer obtainable by esterifying at least one aliphatic carboxylic acid containing three to five carboxyl groups with at least one of the water-soluble polyethers of component II having preferably a number-average molecular weight of ≤ 2000 g/mol, or with a mixture of polyethers containing at least one water-soluble polyether of component II having preferably a number-average molecular weight of ≤ 2000 g/mol,
it being necessary for the sum of the % by weight of components I to IV always to be 100% by weight, and the % by weight of components I-IV being based in each case on the total weight of components I-IV.

2. A mixture according to Claim 1, **characterized in that** it comprises
30% to 70% by weight of component I,
2% to 10% by weight of component II,
10% to 50% by weight of component III, and
5% to 20% by weight of component IV,
it being necessary for the sum of the % by weight of components I to IV always to be 100% by weight, and the % by weight of components I to IV being based in each case on the total weight of components I to IV.

3. A mixture according to Claim 1 or 2, **characterized in that** component I is at least 75% salified and preferably is in the form of an alkali metal, alkaline earth metal or ammonium salt, more preferably in the form of a sodium or potassium salt.

4. A mixture according to any of Claims 1 to 3, **characterized in that** the salified copolymer of component I has been prepared by polymerizing at least one ethylenically unsaturated, phenyl-group-containing monomer having preferably 8 to 20 C atoms, it being possible for the phenyl radicals to be substituted optionally at least once, and at least one α,β-unsaturated monocarboxylic acid having preferably 3 to 8 carbon atoms and/or at least one α,β-unsaturated dicarboxylic acid having preferably 4 to 10 carbon atoms and/or the cyclic anhydrides thereof, and optionally the respective derivatives thereof, preferably esters and amides.

5. A mixture according to Claim 4, **characterized in that** at least one monomer selected from the group encompassing styrene, benzyl (meth)acrylate, and phenyl acrylate, it being possible for the phenyl radicals to be substituted optionally 1 to 4 times, preferably optionally substituted styrene, has been used as said ethylenically unsaturated, phenyl-group-containing monomer, and
acrylic acid or methacrylic acid has been used as α,β-unsaturated monocarboxylic acid, and at least one dicarboxylic acid selected from the group encompassing maleic acid, fumaric acid, and itaconic acid and/or maleic anhydride, and optionally, as respective derivative thereof, preferably ester or amide, has been used as α,β-unsaturated dicarboxylic acid and/or cyclic anhydride thereof.

6. A mixture according to any of Claims 1 to 5, **characterized in that** component I is a salified styrene/maleic anhydride copolymer having a styrene to maleic anhydride ratio of 1:1 to 8:1, preferably of 1:1 to 2:1.

7. A mixture according to any of Claims 1 to 6, **characterized in that** component II is at least one water-soluble polyether synthesized to an extent of at least 50 mol% from ethylene oxide.

8. A mixture according to any of Claims 1 to 7, **characterized in that** component II is at least one water-soluble polyether synthesized from units of ethylene oxide and from units of at least one other alkylene oxide, preferably propylene oxide, butylene oxide and/or styrene oxide or
is synthesized from ethylene oxide units and from units of at least one aliphatic or aromatic glycidyl ether having 3 to 20 C atoms, preferably isopropyl glycidyl ether, n-butyl glycidyl ether, phenyl glycidyl ether or 2-ethylhexyl polyglycidyl ether, and/or from units of at least one aliphatic lactone having 3 to 10 C atoms, preferably propiolactone, valerolactone and/or ε-caprolactone.

9. A mixture according to any of Claims 1 to 8, **characterized in that** component III is the esterification product of at least one water-soluble polyether of component II or of a mixture of polyethers which comprises at least one water-soluble polyether of component II and of an aliphatic, optionally saturated dimer acid which is obtainable by oligomerizing unsaturated fatty acids having 12 to 22 C atoms.

10. A mixture according to any of Claims 1 to 9, **characterized in that** component III is the esterification product of at least one water-soluble polyether of component II or of a mixture of polyethers which comprises at least one water-soluble polyether of component II and
of an optionally partially esterified, aliphatic dicarboxylic acid which is obtainable by reacting an optionally unsaturated, aliphatic dicarboxylic anhydride, preferably maleic anhydride or succinic anhydride, with at least one aliphatic diol with C₂ to C₂₀, which may optionally be chain-extended as a result of reaction with a dihydroxy-terminated, aliphatic polyester based on at least one aliphatic lactone having 3 to 10 C atoms, or
of an optionally partially amidated, aliphatic dicarboxylic acid which is obtainable by reacting an optionally unsaturated, aliphatic dicarboxylic anhydride, preferably maleic anhydride or succinic anhydride, with at least one linear or branched, aliphatic or cycloaliphatic diamine containing two primary amino groups and having 2 to 20 C atoms.

11. A mixture according to any of Claims 1 to 10, **characterized in that** component IV is the esterification product of at least one of the water-soluble polyethers of component II or of a mixture of polyethers which comprises at least one water-soluble polyether of component II and of an optionally saturated trimer acid which is obtainable by oligomerizing an unsaturated fatty acid having 12 to 22 C atoms.

12. A mixture according to any of Claims 1 to 11, **characterized in that** component IV is the esterification product of at least one water-soluble polyether of component II or of a mixture of polyethers which comprises at least one water-soluble polyether of component II, and
of at least one aliphatic, optionally partially esterified multicarboxylic acid which contains 3 to 5 carboxyl groups and is obtainable by reacting an optionally unsaturated, aliphatic dicarboxylic anhydride, preferably maleic anhydride or succinic anhydride, with at least one aliphatic polyol having 3 to 5 OH groups, which is optionally chain-extended as a result of reaction with a polyester based on aliphatic lactones having 3 to 10 C atoms, in a stoichiometric ratio of the anhydride function to the hydroxyl function of 1:1, or
of an aliphatic, optionally amidated multicarboxylic acid which contains 3 to 5 carboxyl groups and is obtainable by reacting an optionally unsaturated, aliphatic dicarboxylic anhydride, preferably maleic anhydride or succinic anhydride, with aliphatic polyamines having a total of 3 to 5 primary and/or secondary amino groups, in a stoichiometric ratio of the anhydride function to the amino function of 1:1.

13. A mixture according to any of Claims 1 to 11, **characterized in that** component III and IV is the esterification product of a mixture of dimer acid and trimer acid, which is obtainable by oligomerizing unsaturated fatty acids having 12 to 22 C atoms, with at least one of the water-soluble polyethers of component II or with a mixture of polyethers which comprises at least one water-soluble polyether of component II, where preferably the ratio of dimer acid to trimer acid in the mixture used is 10:90 to 90:10 and preferably the dimer acid has up to 36 C atoms and the trimer acid has up to 54 C atoms.

14. Use of a mixture according to any of Claims 1 to 13 as a low-VOC wetting and dispersing agent, where the fraction of volatile organic solvents must not exceed the limits defined according to ISO 11890-2 or DIN 55649.

15. Use according to Claim 14 for dispersing solids, preferably for dispersing pigments, or for preparing a low-VOC, aqueous pigment concentrate, where the fraction of volatile organic solvents must not exceed the limits defined according to ISO 11890-2 or DIN 55649.

16. Use of a pigment concentrate according to Claim 15 for producing coating materials, preferably paints.

17. Aqueous, low-VOC pigment concentrate comprising as wetting and dispersing agent a mixture according to any of Claims 1 to 13, where the fraction of volatile organic solvents must not exceed the limits defined according to ISO 11890-2 or DIN 55649.

18. A paint or a paste comprising at least one pigment, a mixture according to any of Claims 1 to 13, an aqueous dispersion medium, and optionally a low-VOC, organic vehicle, where the fraction of volatile organic solvents must not exceed the limits defined according to ISO 11890-2 or DIN 55649, and optionally other auxiliaries.

19. A dispersible, multiparticulate and/or fibrous solid, preferably a pigment, coated with a mixture according to any of Claims 1 to 13.

20. Use of a mixture according to any of Claims 1 to 13 for producing an ink, preferably an aqueous ink for the nonimpact printing process, or as a wetting and dispersing agent in cosmetic preparations, preferably W/O or O/W emulsions, solutions, gels, creams, lotions or sprays.

## Revendications

1. Mélange pauvre en composés organiques volatils (COV), dont la teneur en solvants organiques très volatils ne doit pas excéder les valeurs limites établies selon ISO 11890-2 ou DIN 55649, composé de
I 10 à 80 % en poids d'un copolymère, salifié à raison d'au moins 50 %, d'au moins un monomère à insaturation éthylénique, contenant des groupes phényle, et d'au moins un acide monocarboxylique α,β-insaturé et/ou d'au moins un acide dicarboxylique α,β-insaturé et éventuellement de leurs dérivés,
II 1 à 30 % en poids d'au moins un polyéther linéaire hydrosoluble, comportant un groupe OH terminal, qui est constitué à raison d'au moins 25 % en moles d'oxyde d'éthylène,
III 5 à 80 % en poids d'au moins un produit d'estérification d'un acide dicarboxylique aliphatique et d'au moins l'un des polyéthers hydrosolubles du composant II ayant de préférence une masse moléculaire moyenne en nombre de ≤ 2 000 g/mole ou d'un mélange de polyéthers qui comporte au moins un polyéther hydrosoluble du composant II ayant de préférence une masse moléculaire moyenne en nombre de ≤ 2 000 g/mole,
et
IV 3 à 80 % en poids d'au moins un polymère en étoile pouvant être obtenu par estérification d'au moins un acide carboxylique aliphatique, comportant de trois à cinq groupes carboxy, avec au moins l'un des polyéthers hydrosolubles du composant II, ayant de préférence une masse moléculaire moyenne en nombre de ≤ 2 000 g/mole, ou d'un mélange de polyéthers ayant de préférence une masse moléculaire moyenne en nombre de ≤ 2 000 g/mole,
la somme des % en poids des composants I à IV devant toujours être égale à 100 % en poids et les % en poids des composants I-IV étant chacun par rapport au poids total des composants I-IV.

2. Mélange selon la revendication 1, **caractérisé en ce qu'**il contient
30 à 70 % en poids du composant I,
2 à 10 % en poids du composant II,
10 à 50 % en poids du composant III et
5 à 20 % en poids du composant IV,
la somme des % en poids des composants I à IV devant toujours être égale à 100 % en poids et les % en poids des composants I-IV étant chacun par rapport au poids total des composants I-IV.

3. Mélange selon la revendication 1 ou 2, **caractérisé en ce que** le composant I est salifié à raison d'au moins 75 % et se trouve de préférence sous forme de sel de métal alcalin, de métal alcalinoterreux ou d'ammonium, de façon particulièrement préférée sous forme de sel de sodium ou de potassium.

4. Mélange selon l'une quelconque des revendications 1 à 3, **caractérisé en ce que** le copolymère salifié du composant I a été préparé par polymérisation d'au moins un monomère à insaturation éthylénique, contenant le groupe phényle, ayant de préférence de 8 à 20 atomes de carbone, les radicaux phényle pouvant éventuellement être au moins monosubstitués, et d'au moins un acide monocarboxylique α,β-insaturé ayant de préférence de 3 à 8 atomes de carbone et/ou d'au moins un acide dicarboxylique α,β-insaturé ayant de préférence de 4 à 10 atomes de carbone et/ou de leurs anhydrides cycliques et éventuellement de leurs dérivés respectifs, de préférence esters et amides.

5. Mélange selon la revendication 4, **caractérisé en ce que** comme monomère à insaturation éthylénique, contenant le groupe phényle, on a utilisé au moins un monomère choisi dans le groupe comprenant le styrène, le (méth)acrylate de benzyle et l'acrylate de phényle, les radicaux phényle pouvant éventuellement être 1 à 4 fois substitués, de préférence un styrène éventuellement substitué, et
on a utilisé comme acide monocarboxylique α,β-insaturé l'acide acrylique ou l'acide méthacrylique et comme acide dicarboxylique α,β-insaturé et/ou son anhydride cyclique au moins un acide dicarboxylique choisi dans le groupe comprenant l'acide maléique, l'acide fumarique et l'acide itaconique et/ou l'anhydride maléique, et éventuellement sous forme de son dérivé respectif, de préférence un ester ou amide.

6. Mélange selon l'une quelconque des revendications 1 à 5, **caractérisé en ce que** le composant I est un copolymère styrène/anhydride maléique salifié ayant un rapport styrène à anhydride maléique de 1:1 à 8:1, de préférence de 1:1 à 2:1.

7. Mélange selon l'une quelconque des revendications 1 à 6, **caractérisé en ce que** le composant II est constitué d'au moins un polymère hydrosoluble, qui est constitué au moins à raison de 50 % en moles d'oxyde d'éthylène.

8. Mélange selon l'une quelconque des revendications 1 à 7, **caractérisé en ce que** le composant II est au moins un polyéther hydrosoluble qui est constitué de motifs de l'oxyde d'éthylène et de motifs d'au moins un autre oxyde d'alkylène, de préférence l'oxyde de propylène, l'oxyde de butylène et/ou l'oxyde de styrène ou
de motifs d'oxyde d'éthylène et de motifs d'au moins un éther glycidique aliphatique ou aromatique ayant de 3 à 20 atomes de carbone, de préférence l'éther isopropylglycidique, l'éther n-butylglycidique, l'éther, phénylglycidique ou l'éther 2-éthylhexylpolyglycidique, et/ou de motifs d'au moins une lactone aliphatique ayant de 3 à 10 atomes de carbone, de préférence la propionolactone, la valérolactone et/ou l'ε-caprolactone.

9. Mélange selon l'une quelconque des revendications 1 à 8, **caractérisé en ce que** le composant III est le produit d'estérification d'au moins un polyéther hydrosoluble du composant II ou d'un mélange de polyéthers qui contient au moins un polyéther hydrosoluble du composant II, et d'un acide dimère aliphatique éventuellement saturé qui peut être obtenu par oligomérisation d'acides gras insaturés ayant de 12 à 22 atomes de carbone.

10. Mélange selon l'une quelconque des revendications 1 à 9, **caractérisé en ce que** le composant III est le produit d'estérification d'au moins un polyéther hydrosoluble du composant II ou d'un mélange de polyéthers qui contient au moins un polyéther hydrosoluble du composant II, et
d'un acide dicarboxylique aliphatique éventuellement estérifié, qui peut être obtenu par la réaction d'un anhydride dicarboxylique aliphatique éventuellement insaturé, de préférence l'anhydride maléique ou succinique, avec au moins un diol aliphatique en C₂ à C₂₀, dont la chaîne peut éventuellement être prolongée par mise en réaction avec un polyester aliphatique à terminaison dihydroxy, à base d'au moins une lactone aliphatique ayant de 3 à 10 atomes de carbone, ou
d'un acide dicarboxylique aliphatique éventuellement en partie amidé, qui peut être obtenu par la réaction d'un anhydride dicarboxylique aliphatique éventuellement insaturé, de préférence l'anhydride maléique ou succinique, avec au moins une diamine linéaire ou ramifiée, aliphatique ou cycloaliphatique, comportant deux groupes amino primaires, ayant de 2 à 20 atomes de carbone.

11. Mélange selon l'une quelconque des revendications 1 à 10, **caractérisé en ce que** le composant IV est le produit d'estérification d'au moins un polyéther hydrosoluble du composant II ou d'un mélange de polyéthers qui contient au moins l'un des polyéthers hydrosolubles du composant II, et d'un acide trimère éventuellement saturé qui peut être obtenu par oligomérisation d'un acide gras insaturé ayant de 12 à 22 atomes de carbone.

12. Mélange selon l'une quelconque des revendications 1 à 11, **caractérisé en ce que** le composant IV est le produit d'estérification d'au moins un polyéther hydrosoluble du composant II ou d'un mélange de polyéthers qui contient au moins un polyéther hydrosoluble du composant II, et
d'au moins un acide polycarboxylique aliphatique éventuellement en partie estérifié, comportant de 3 à 5 groupes carboxy, qui peut être obtenu par la réaction d'un anhydride d'acide dicarboxylique aliphatique éventuellement insaturé, de préférence l'anhydride maléique ou succinique, avec au moins un polyol aliphatique comportant 3 à 5 groupes OH, dont la chaîne peut éventuellement être prolongée par mise en réaction avec un polyester à base de lactones aliphatiques ayant de 3 à 10 atomes de carbone, en un rapport stoechiométrique de la fonction anhydride à la fonction hydroxy de 1:1, ou
d'un acide polycarboxylique aliphatique éventuellement amidé, comportant de 3 à 5 groupes carboxy, qui peut être obtenu par la réaction d'un anhydride d'acide dicarboxylique aliphatique éventuellement insaturé, de préférence l'anhydride maléique ou succinique, avec des polyamines aliphatiques comportant au total 3 à 5 groupes amino primaires et/ou secondaires, en un rapport stoechiométrique de la fonction anhydride à la fonction amino de 1:1.

13. Mélange selon l'une quelconque des revendications 1 à 11, **caractérisé en ce que** les composants III et IV consistent en le produit d'estérification d'un mélange d'acide dimère et d'acide trimère, pouvant être obtenus par oligomérisation d'acides gras insaturés ayant de 12 à 22 atomes de carbone, avec au moins l'un des polyéthers hydrosolubles du composant II ou un mélange de polyéthers qui contient au moins un polyéther hydrosoluble du composant II, de préférence le rapport de l'acide dimère à l'acide trimère dans le mélange utilisé valant de 10:90 à 90:10 et de préférence l'acide dimère ayant jusqu'à 36 atomes de carbone et l'acide trimère ayant jusqu'à 54 atomes de carbone.

14. Utilisation d'un mélange selon l'une quelconque des revendications 1 à 13, en tant qu'agent mouillant et dispersant pauvre en COV, dont la teneur en solvants organiques très volatils ne doit pas excéder les valeurs limites établies selon ISO 11890-2 ou DIN 55649.

15. Utilisation selon la revendication 14, pour la dispersion de solides, de préférence pour la dispersion de pigments, ou pour la préparation d'un concentré aqueux de pigment, pauvre en COV, dont la teneur en solvants organiques très volatils ne doit pas excéder les valeurs limites établies selon ISO 11890-2 ou DIN 55649.

16. Utilisation d'un concentré de pigment selon la revendication 15, pour la production de matières de revêtement, peintures de préférence.

17. Concentré aqueux de pigment, pauvre en COV, contenant en tant qu'agent mouillant et dispersant un mélange selon l'une quelconque des revendications 1 à 13, dont la teneur en solvants organiques très volatils ne doit pas excéder les valeurs limites établies selon ISO 11890-2 ou DIN 55649.

18. Peinture ou pâte contenant au moins un pigment, un mélange selon l'une quelconque des revendications 1 à 13, un milieu aqueux de dispersion et éventuellement un médium organique pauvre en COV, dont la teneur en solvants organiques très volatils ne doit pas excéder les valeurs limites établies selon ISO 11890-2 ou DIN 55649, et éventuellement d'autres adjuvants.

19. Corps solide dispersable, multiparticulaire et/ou fibreux, de préférence un pigment, enrobé avec un mélange selon l'une quelconque des revendications 1 à 13.

20. Utilisation d'un mélange selon l'une quelconque des revendications 1 à 13, pour la fabrication d'une encre, de préférence d'une encre aqueuse pour le procédé d'impression « non impact », ou en tant qu'agent mouillant et dispersant dans des préparations cosmétiques, de préférence des émulsions E/H ou H/E, solutions, gels, crèmes, lotions ou compositions à pulvériser en aérosol.
